Europäisches Patentamt

European Patent Office

Office européen des brevets

(19)

(11) Veröffentlichungsnummer : **0 096 840**
**B1**

(12) **EUROPÄISCHE PATENTSCHRIFT**

(45) Veröffentlichungstag der Patentschrift :
25.03.87

(21) Anmeldenummer : 83105620.5

(22) Anmeldetag : 08.06.83

(51) Int. Cl.⁴ : **G 01 N 31/00**, G 01 N 27/56,
G 01 N 33/84, G 01 N 21/64

(54) Verfahren und Anordnung zur Messung der Ionenstärke.

(30) Priorität : 14.06.82 DE 3222325

(43) Veröffentlichungstag der Anmeldung :
28.12.83 Patentblatt 83/52

(45) Bekanntmachung des Hinweises auf die Patenterteilung : 25.03.87 Patentblatt 87/13

(84) Benannte Vertragsstaaten :
AT BE CH FR GB IT LI LU NL SE

(56) Entgegenhaltungen :
DE-A- 2 929 387
G. KORTÜM "Lehrbuch der Elektrochemie", 4.
Auflage, 1966 VERLAG CHEMIE GMBH, Wein-
heim/Bergstr., Seiten 338-343
ULLMANNS ENCYCLOPÄDIE DER TECHNISCHEN
CHEMIE, 4. auflage, Band 13, 8 1977, VERLAG CHE-
MIE, Weinheim, New York, Seiten 184, 189, 190
Die Akte enthält technische Angaben, die nach dem
Eingang der Anmeldung eingereicht wurden und die
nicht in dieser Patentschrift enthalten sind.

(73) Patentinhaber : Max-Planck-Gesellschaft zur Förderung der Wissenschaften e.V.
Bunsenstrasse 10
D-3400 Göttingen (DE)

(72) Erfinder : Lübbers, Dietrich Werner, Prof. Dr.
Rheinlanddamm 201 a
D-4600 Dortmund 1 (DE)
Erfinder : Opitz, Norbert, Dr. Dipl. Phys.
Villigsterstrasse 6
D-5840 Schwerte (DE)

(74) Vertreter : Hofmann, Hans Walter, Dr.
Hauberisserstrasse 36
D-6200 Wiesbaden (DE)

**Beschreibung**

Die Erfindung betrifft ein Verfahren und eine Anordnung zur Messung der Ionenstärke.
Die Ionenstärke J wird durch

$$J = \frac{1}{2} \sum_i C_i W_i^2 \qquad (1)$$

$C_i$ = Konzentration der Spezies i
$W_i$ = Wertigkeit der Spezies i

definiert.

Soll sie bestimmt werden, muss danach die Konzentration der einzelnen Spezies gemessen und aus den üblicherweise tabellarisch erfassten elektrochemischen Wertigkeiten der Spezies die Ionenstärke nach (1) berechnet werden.

Für die meisten Mess- und Interpretationsprobleme ist eine derartige Bestimmung der Ionenstärke ausreichend.

Bei biologischen Objekten aber, beispielsweise bei Stoffwechselvorgängen, treten häufig zeitliche Änderungen der Stoffzusammensetzung in den Elektrolyten auf. Sollen jetzt die jeweiligen Ionenstärken bestimmt werden, ist der mühevolle Weg über die Bestimmung der Fraktionen der beteiligten Stoffe sowie deren Wertigkeiten und Konzentration und die Rückrechnung über die Tabellen meist in der zur Verfügung stehenden kurzen Prozesszeit nicht möglich.

Die unmittelbare Messung der Ionenstärke von Elektrolyten hat sich die Erfindung deshalb zur Aufgabe gemacht.

Sie löst diese Aufgabe dadurch, dass der pH-Wert des Elektrolyten mittels einer ersten Messung mit einem ersten, von der Ionenstärke abhängigen Indikator und einem zweiten, von der Ionenstärke in vom ersten Indikator verschiedener Weise abhängigen Indikator gemessen wird, wobei die Ionenstärke vermittels der Näherung

$$J = J_0 \cdot 10^{\; F(N)\left[ 1 + \frac{W(X) + 0,5}{W(N) - W(X)} \right] [pH'(N) - pH'(X)]} \qquad (2)$$

$J$ = Ionenstärke
$J_0$ = Ionenstärke bei Eichung
$pH'(N)$ = pH-Wert, gemessen mit einem ersten, von der Ionenstärke abhängigen Indikator N (HPTS)
$pH'(X)$ = pH-Wert, gemessen mit einem zweiten, von der Ionenstärke abhängigen Indikator X, dessen Ionenstärkenabhängigkeit verschieden ist von der des ersten Indikators
$W(X)$ = Wertigkeit des ersten Indikators
$W(N)$ = Wertigkeit des zweiten Indikators (—4)

mit

$$F(N) = -\frac{1}{0,07(2\,W(N) + 1)} \qquad (2a)$$

errechnet wird.

Der Vorteil des erfindungsgemässen Verfahrens liegt darin, dass nunmehr die Ionenstärke auch von Mischungen an sich unbekannter Zusammensetzung unmittelbar bestimmbar ist.

Eine Anordnung zur Ausführung des Verfahrens wird in Anspruch 2 beschrieben.

Das Verfahren kann mit Fluoreszenzindikatoren, Absorptionsindikatoren, Elektroden als Indikatoren oder sonstigen anzeigenden Verfahren durchgeführt werden, wenn nur die beiden Messungen eine unterschiedliche Abhängigkeit von der Ionenstärke aufweisen. Die Elektroden können Glaselektroden oder ionenselektive Elektroden, beispielsweise für die pH-Messung, ionenselektive oder Glaselektroden für die Messung von Ca-Ionen sein. Zur fluorezenzfotometrischen Messung von Calziumionen kann Arsenazo-III® als Absorptionsindikator verwendet werden.

Ein besonderes Verfahren ist dabei das Doppelindikatorverfahren, nämlich die Messung mit zwei verschiedenen Fluorszenzindikatoren, weil deren Fluoreszenzsignale messtechnisch gut separierbar sind.

Für physiologische Messungen, insbesondere an Blut und Harn, ist die Verwendung von Hydroxypyrentrisulfonat (HPTS) und b-Methylumbelliferon (b-M) besonders vorteilhaft.

Weiterhin kann den Fluoreszenzindikatoren zur Ausschaltung von Intensitätsschwankungen in bekannter Weise ein Referenzindikator zugeordnet sein.

Für den Fall, dass der pH-Wert mittels einer fluoreszensfotometrischen Messung und einer pH-Messung mit der pH-Glaselektrode als Indikator bestimmt werden soll, wird die Ionenstärke mittels der Beziehung

$$J = J_0 \cdot 10^{2.22[pH'(N) - pH]} \tag{4}$$

pH'(N) = pH-Wert mit einem ersten Indikator N (HPTS) gemessen
pH = pH-Wert mit einer pH-Elektrode gemessen

berechnet.

Die Anordnung zur Ausführung dieses Verfahrens wird in Anspruch 7 beschrieben.

Die Wahl der Messmittel soll derart erfolgen, dass die Signaldifferenz einen möglichst grossen Wert hat. Insoweit ist die Verwendung von pH-Elektroden oder von selektiven Elektroden vorteilhaft, weil die Elektroden eine nur geringe Abhägigkeit von der Ionenstärke aufweisen. Dadurch wird die Signaldifferenz, mit der potenziert werden muss, besonders gross und damit ist auch das Messignal besonders gross. Dieser Vorteil wird noch weiter dadurch verbessert, dass die Wertigkeit des verwendeten ersten Indikators möglichst hoch ist.

Bei den beschriebenen Messmethoden und Anordnungen ist die Verwendung von HPTS als Fluorszenzindikator für die pH-Messung an sich willkürlich. Jeder andere Fluoreszenzindikator ist ebenso verwendbar. Allerdings ändert sich dann der Exponentenfaktor (der für den Indikator HPTS etwa 2.22 beträgt).

Der einem beliebigen Indikator (X) zugeordnete Exponentenfaktor F(X) ist ohne weiteres bestimmbar. Es ist

$$F(X) = F(N) \frac{Z(N)}{Z(X)} \tag{6}$$

F(N) = Indikatorspezifische Exponentenkonstante für N (HPTS)
F(X) = Exponentenkonstante für Indikator X
Z(N) = Wertigkeitsparameter für N (HPTS)
Z(X) = Wertigkeitsparameter für Indikator X
W = Wertigkeit

mit

$$Z = Z(-W)-1 \tag{7}$$

Mit Anordnungen dieser Art ist durch Umrechnung weiterhin der osmotische Druck oder die Siedpunktserhöhung bestimmbar. Für den osmotischen Druck Po beispielsweise ist :

$$P_0 \approx Z/J/ \cdot 22.4 \text{ atm} \tag{8}$$

1 atm ≈ 1 bar

und für die Osmolarität ist :

$$Osm \approx 2J \tag{9}$$

Das erfindungsgemässe Verfahren lässt sich weiterhin dazu verwenden, den pH-Wert einer Lösung mit unbekannter Ionenstärke oder darüberhinaus von unbekannten Lösungen von Elektrolyten nunmehr auch fluoreszenzfotometrisch zu bestimmen.

Wird nämlich ein fluoreszierender pH-Indikator fotometriert, dann zeigt sich, dass der gemessene pH-Wert je nach der Ionenstärke des zu messenden Elektrolyten vom tatsächlichen pH-Wert abweicht. Zur Elimination dieser Abweichung wird der pH-Wert mit zwei verschiedenen Indikatoren unterschiedlicher Abhängigkeit von der Ionenstärke gemessen und der tatsächliche pH-Wert mittels der Beziehung.

$$pH = pH'(X) - \frac{W(X) + 0{,}5}{W(N) - W(X)} [pH'(N) - pH'(X)] \tag{10}$$

pH'(N) = scheinbarer pH(N)-Wert
pH'(X) = scheinbarer pH(X)-Wert
pH = tatsächlicher pH-Wert

3

bestimmt.

Eine solche optische Methode der pH-Messung lässt sich, weil sie kontinuierlich erfolgen kann, beispielsweise vorteilhaft zur Prozessteuerung verwenden.

Weiterhin lässt sich die Messung in explosionsgefährdeten Räumen verwenden, da sie beispielsweise über Lichtleiter vorgenommen werden kann. Dadurch können alle elektrischen Potentiale, beispielsweise die Versorgungsspannungen der Messeinrichtung, in einem solchen Raum vermieden werden.

Die Messmethode wird nunmehr wie folgt erläutert:

Ausgangspunkt ist das Massenwirkungsgesetz in der Form

$$pH = pK - \text{Log}\left(\frac{Sd - Sx}{Sx}\right) = pK - \text{Log}\left(\frac{1 - \alpha}{\alpha}\right) \tag{11}$$

mit

Sd = Konzentration des vollständig dissoziierten Elektrolyten
Sx = Konzentration des aktuell dissoziierten Teils des Elektrolyten
$\alpha$ = Sx/Sd = Dissoziationsgrad des Elektrolyten

Da, wie sich experimentell gezeigt hat, die Verschiebung des fluoreszenzfotometrisch gemessenen pH-Wertes proportional

$$\Delta pK = pK_1 - pK_2 \tag{12}$$

ist, mit

pK1 = pK-Wert bei Ionenstärke 1
pK2 = pK-Wert bei Ionenstärke 2

ergibt sich bei zwei Messungen derselben Lösung mit verschiedenen pH-Indikatoren unterschiedlicher Abhängigkeit der Ionenstärke das Gleichungssystem

$$pH = pH'(N) - \Delta pK(N)$$

$$pH = pH'(X) - \Delta pK(X) \tag{13}$$

In den Gleichungen (13) sind die Grössen

$$pH, \Delta pK(N), \Delta pK(X)$$

unbekannt. Das insoweit nicht auflösbare System (13) lässt sich aber dann lösen, wenn beachtet wird, dass nach DEBYE-HÜCKEL näherungsweise eine Kopplung der Abweichungen

$$\Delta pK(N), \Delta pK(X)$$

besteht. Es ist nämlich nach DEBYE-HÜCKEL

$$\Delta pK = Z \cdot L(J) \tag{14}$$

mit

$$Z = 2(-W) - 1 \tag{15}$$

W = Wertigkeit des Indikators

und der DEBYE-HÜCKEL-Funktion

$$L(J) = A\frac{\sqrt{J}}{1 + B \cdot d \cdot \sqrt{J}} \tag{16}$$

mit

J = Ionenstärke
B,d = Molekülparameter

A = Temp. abh. Konst

Damit ergeben sich aus (13) mit der Näherung

$$L(J,X) \approx L(J,N) = L(J) \tag{17}$$

Die Gleichungen

$$\Delta pK(N) = Z(N) \cdot L(J)$$

$$\Delta pK(X) = Z(X) \cdot L(J) \tag{18}$$

Es besteht also die Kopplung

$$\Delta pK(N) = \frac{Z(N)}{Z(X)} \Delta pK(X) . \tag{19}$$

Aus (13) und (19) folgt

$$pH = \frac{1}{Z(X) - Z(N)} [Z(X) \, pH'(N) - Z(N) \, pH'(X)] \tag{20}$$

beziehungsweise mit den Wertigkeiten W

$$pH = pH'(X) - WF[pH'(N) - pH'(X)] \tag{21}$$

mit

$$WF = \frac{W(X) + 0{,}5}{W(N) - W(X)} . \tag{21a}$$

Experimentell ergeben sich die folgenden Kurven:
Für beispielsweise HPTS ergibt sich:

$$\Delta pK(HPTS) = 0{,}45 \, Log \frac{J}{J_0} \tag{22}$$

für beispielsweise b-Methylumbelliferon (b-M) ergibt sich:

$$\Delta pK(b - M) = 0{,}07 \, Log \frac{J}{J_0} \tag{23}$$

im praktisch wichtigsten Ionenstärkebereich

$$J_0 < J < 10 J_0 \; ; \; J_0 = 0{,}1$$

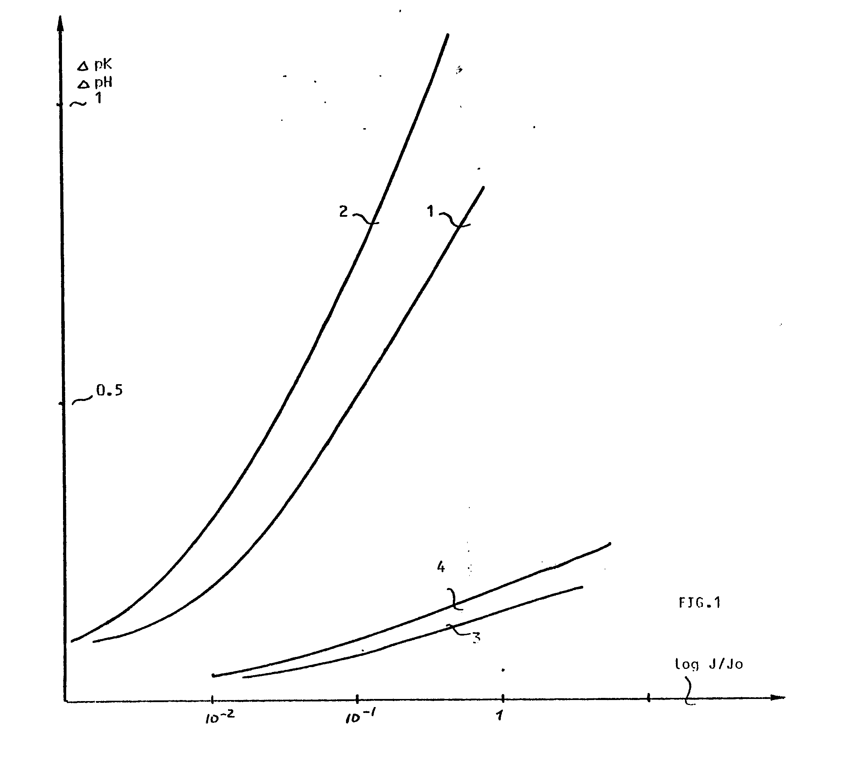

die in Fig. 1 dargestellt sind. Es ist 1 die gemessene, 2 die nach DEBYE-HÜCKEL zu erwartende Kurve für HPTS, 3 die gemessene, 4 die nach DEBYE-HÜCKEL theoretisch zu erwartende Kurve für b-M.
Umgerechnet ergibt sich aus den Gleichungen (22), (23) die Ionenstärke bei Messung der pH-Werte mittels pH-Elektrode und mittels Fluoreszenzfotometrie (Indikator HPTS)

$$J = J_0 \cdot 10^{1/0{,}45(pH'(N) - pH)} = J_0 \cdot 10^{2{,}22(pH'(N) - pH)} \tag{24}$$

und bei Messung mit zwei Fluoreszenzindikatoren, etwa HPTS und b-Methylumbelliferon, zu

$$J = J_0 \cdot 10^{2{,}22(1 + WF)(p'H(HPTS) - pH'(b - M))}$$

mit

$$\tag{25}$$

$$WF = \frac{W(X) + 0{,}5}{W(N) - W(X)} = \frac{W(b - M) + 0{,}5}{W(HPTS) - W(b - M)} = \frac{-1 + 0{,}5}{-4 + 1} = \frac{1}{6} .$$

Für beliebige Indikatoren X gilt

5

$$J = J_0 \cdot 10^{\;F(N)\left[1 + \frac{W(X) + 0.5}{W(HPTS) - W(X)}\right][pH'(HPTS) - pH'(X)]} \qquad (26)$$

mit

$$F(N) = -\frac{1}{0{,}07(Z(N))} \qquad (26a)$$

und

$$Z(N) = Z(-W(N)) - 1 \qquad (26b)$$

Dabei kann F(N) folgendermassen ermittelt werden :

Die Gleichung (24) entspricht der Gleichung (2). Der Faktor F = 2.22 in Gleichung (24) ist also etwa der Kehrwert der Steigung 0.45 der Kurve (22). Die Steigung wird zweckmässig experimentell bestimmt, kann im Prinzip aber auch theoretisch nach DEBYE-HÜCKEL ermittel werden.

Die Normierung ist dabei willkürlich und es kann auch auf jeden anderen Indikator normiert werden.

Die Ableitung gilt sinngemäss für beliebige Ionen, da Eigenschaften einer bestimmten Ionenart nicht vorausgesetzt werden müssen. Die zur Messung einer bestimmten Ionenart erforderlichen beiden Indikatoren müssen jedoch in unterschiedlicher Weise von der Ionenstärke der Lösung abhängen.

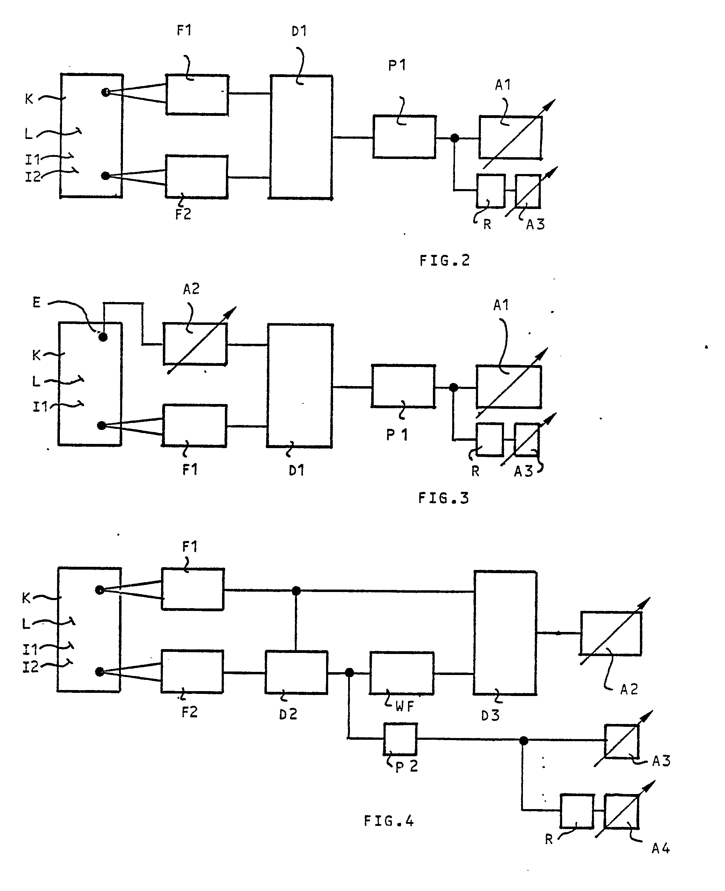

Fig. 2 und Fig. 3 zeigen einfache Anordnungen, mit denen die beschriebene Methode ausgeführt werden kann.

In Fig. 2 bestimmen zwei auf die pH-sensitiven Indikatoren I1, I2 eingestellten Fluoreszenzfotometer F1, F2 den pH-Wert derselben Lösung L in einer Küvette K. Die beiden pH-Werte werden elektrisch einem Subtrahierglied D1 zugeführt und mit der dort gebildeten Differenz d1 wird in einem Potenzierglied P1 die Größe.

$$E_1 = J_0 \cdot 10^{\;F(N)\left[1 + \frac{W(X) + 0.5}{W(N) - W(X)}\right]} \qquad (3)$$

mit

$$pH'(N) - pH'(X) \qquad (3a)$$

potenziert.

Der im Potenzierglied, das als Operationsverstärker oder als Digitalrechner ausgebildet sein kann, gebildete Wert wird in einer Anzeige A1 angezeigt.

Parallel zur Anzeige der Ionenstärke kann in einer Rechenvorrichtung R eine Umrechnung des Signals erfolgen, sodass in einer Anzeigevorrichtung A3 gleichzeitig weitere ableitbare Signale angezeigt werden können. Erfolgt in R beispielsweise eine Multiplikation mit 2, dann zeigt die Anzeigevorrichtung die Osmolarität gemäss Gleichung (9) an, ist der Faktor die Grösse 44.8, dann zeigt A3 den osmotischen Druck gemäss Gleichung (8) an.

Andere Rechenvorschriften, die von der Ionenstärke abhängen können ebenfalls verwendet werden.

In Fig. 3 ist anstelle des Fotometers F2 und des Indikators I2 die Elektrode E getreten. In einer solchen Anordnung kann leicht zusätzlich des pH-Wert durch eine Anzeigevorrichtung A2 angezeigt werden. Damit ist beispielsweise bei Stoffwechselvorgängen der Hinweis auf Änderungen der Stoffzusammensetzung ermöglicht, die sich bei konstantem pH-Wert ergeben kann und die diagnostisch von Bedeutung ist. Die Messung abgeleiteter Grössen erfolgt entsprechend Fig. 2.

In Fig. 4 wird der pH-Wert in der Küvette K mit den beiden Fotometern F1 und F2 gemessen und die Signaldifferenz d2 in dem Subtrahierglied D2 gebildet. Nach Multiplikation mit dem Wertigkeitsfaktor WF wird dieses Signal vom Signal des Indikators I1 noch einmal in D3 subtrahiert und von der Anzeigeeinrichtung A2 angezeigt. Die parallele Messung der Ionenstärke erfolgt dadurch, dass das Differenzsignal D2 dem Potenzierglied P2 zugeführt und nach Potenzierung in einer Anzeigevorrichtung A4 anzeigbar ist.

Entsprechend Fig. 2 und Fig. 3 kann eine Anzeige abgeleiteter Grössen erfolgen.

**Patentansprüche**

1. Verfahren zur Messung der Ionenstärke, dadurch gekennzeichnet, dass der pH-Wert des Elektrolyten mittels einer ersten Messung mit einem ersten, von der Ionenstärke abhängigen Indikator und mittels einer zweiten Messung mit einem zweiten, von der Ionenstärke in vom ersten Indikator verschiedener Weise abhängigen zweiten Indikator gemessen wird, wobei die Ionenstärke vermittels der Näherung

$$J = J_0 \cdot 10^{F(N)\left[1 + \frac{W(X) + 0.5}{W(N) - W(X)}\right][pH'(N) - pH'(X)]} \tag{2}$$

J = Ionenstärke

$J_0$ = Ionenstärke bei Eichung

pH'(N) = pH-Wert, gemessen mit einem ersten, von der Ionenstärke abhängigen Indikator N (HPTS)

pH'(X) = pH-Wert, gemessen mit einem zweiten, von der Ionenstärke abhängigen Indikator X, dessen Ionenstärkenabhängigkeit verschieden ist von der des ersten Indikators

W(N) = Wertigkeit des ersten Indikators

W(X) = Wertigkeit des zweiten Indikators

mit

$$F(N) = - \frac{1}{0,07(2\,W(N) + 1)} \tag{2a}$$

errechnet wird.

2. Anordnung zur Ausfuehrung des Verfahrens nach Anspruch 1, dadurch gekennzeichnet, dass eine an sich bekannte Anordnung zur Bestimmung des pH-Wertes mittels eines ionenstaerkeabhaengigen ersten Indikators (I1, F1), sowie eine an sich bekannte Anordnung mit einem zweiten Indikator (I2, F2) mit einer gegenueber dem ersten Indikator (I1) verschiedenen Abhaengigkeit von der Ionenstaerke vorgesehen ist, deren Signaldifferenz (d1) mittels eines Potenziergliedes (P1, P2) die Groesse

$$E_1 = J_0 \cdot 10^{F(N)\left[1 + \frac{W(X) + 0,5}{W(N) - W(X)}\right]} \tag{3}$$

zu

$$(E_1)^{d_1} = J_0^{d_1} \cdot 10^{F(N)\left[1 + \frac{W(X) + 0,5}{W(N) - W(X)}\right]d_1} \tag{3b}$$

berechnet, wobei

$$F(N) = - \frac{1}{0,07(2\,W(N) + 1)} \tag{3a}$$

und dass das potenzierte Signal einem Anzeigegeraet (A1) zugefuehrt ist.

3. Verfahren nach Anspruch 1, dadurch gekennzeichnet, dass die Indikatoren zwei verschiedene Fluorszenzindikatoren mit unterschiedlicher Abhängigkeit von der Ionenstärke sind.

4. Verfahren nach Anspruch 3, dadurch gekennzeichnet, dass die Indikatoren Hydroxypyrentrisulfonat (HPTS) und b-Methylumbelliferon (b-M) sind.

5. Verfahren nach Anspruch 3, dadurch gekennzeichnet, dass den Fluoreszenzindikatoren zur Ausschaltung von Intensitätsschwankungen in bekannter Weise ein Referenzindikator zugeordnet ist.

6. Verfahren nach Anspruch 1, dadurch gekennzeichnet, dass einer der Indikatoren eine pH-Elektrode ist und dass die Ionenstärke mittels der Beziehung

$$J = J_0 \cdot 10^{2.22[pH'(N) - pH]} \tag{4}$$

pH'(N) = pH-Wert mit einem ersten Indikator N (HPTS) gemessen

pH = pH-Wert mit der pH-Elektrode gemessen

berechnet ist.

7. Anordnung zur Ausfuehrung des Verfahrens nach Anspruch 6, dadurch gekennzeichnet, dass eine an sich bekannte Anordnung zur fluoreszenzfotometrischen ersten Messung des pH-Wertes (I1, F1) und eine pH-Elektrode (E) zur zweiten Messung des pH-Wertes vorgesehen ist, mit deren Signaldifferenz (d1) mittels eines Potenziergliedes (P2)

$$E_2 = J_0 \cdot 10^{2.22} \tag{5}$$

zu

7

**0 096 840**

$$E_2^{d1} = J_0^{d1} \cdot 10^{2{,}22 \cdot d1} \tag{5b}$$

berechnet und das potenzierte Signal einem Anzeigegeraet (A1) zugefuehrt ist.

8. Verfahren zur fluoreszenzfotometrischen Messung des pH-Wertes einer unbekannten Lösung von Elektrolyten, dadurch gekennzeichnet, dass vom mit einem ersten Indikator (X) gemessenen pH-Wert die mit einem Wertigkeitsfaktor (WF) multiplizierte Signaldifferenz aus dem zweiten Indikator (N) und dem ersten Indikator (X) subtrahiert ist, sodass

$$pH = pH' - WF[pH'(N) - pH'(X)] \tag{21}$$

pH'(N) = scheinbarer pH(N)
pH'(X) = scheinbarer pH(X)
pH = tatsächlicher pH
WF = Wertigkeitsfaktor

mit

$$WF = \frac{W(X) + 0{,}5}{W(N) - W(X)} \cdot \tag{21a}$$

W = Wertigkeit

9. Anordnung zur Ausführung des Verfahrens nach Anspruch 8 dadurch gekennzeichnet, dass eine Subtraktionsanordnung (D3) vorgesehen ist, in der von einem Signal für den vom mit einem ersten Indikator (I1, F1) gemessenen pH-Wert die mit einem Wertigkeitsfaktor (WF) multiplizierte Signaldifferenz (d2) aus dem zweiten Indikator (I2) und dem ersten Indikator (I1) subtrahiert ist, wobei der Wertigkeitsfaktor (WF) den Wert

$$WF = \frac{W(X) + 0{,}5}{W(N) - W(X)} \cdot \tag{21a}$$

W = Wertigkeit

aufweist und dass das resultierende Signal einer Anzeigevorrichtung (A2) zugeführt ist.

10. Verfahren zur fluoreszenzfotometrischen Bestimmung des pH-Wertes nach Anspruch 8, dadurch gekennzeichnet, dass der Messwert einem Prozessrechner und einer Anzeigevorrichtung zugeführt ist.

11. Anordnung nach Anspruch 7, dadurch gekennzeichnet, dass das Signal der pH-Elektrode einer weiteren Anzeigevorrichtung (A2) zugeführt ist.

12. Anordnung nach Anspruch 2 oder 7, dadurch gekennzeichnet, dass eine weitere, mit einer Rechenanordnung (R) versehene Anzeigevorrichtung (A3) vorgesehen ist, mit der von der Ionenstärke abhängige Grössen berechenbar und anzeigbar sind.

13. Anordnung nach Anspruch 12, dadurch gekennzeichnet, dass die Rechenvorschrift der Rechenanordnung (R) die Beziehung

$$Osm = 2\,J$$

ist.

14. Anordnung nach Anspruch 12, dadurch gekennzeichnet, dass die Rechenvorschrift der Rechenanordnung (R) die Beziehung

$$P_0 = 2J \cdot 22{,}4$$

ist.

15. Anordnung nach Anspruch 9, dadurch gekennzeichnet, dass die Signaldifferenz (d2) mittels eines Potenziergliedes (P1) die Groesse

$$J_0 \cdot 10^{2{,}22}$$

zu

$$J_1 = J_0^{d2} \cdot 10^{2{,}22 \cdot d2}$$

berechnet und das potenzierte Signal einer weiteren Anzeigevorrichtung (A3) fuer die Ionenstaerke zugefuehrt ist.

8

16. Anordnung nach Anspruch 15, dadurch gekennzeichnet, dass die eine weitere, mit einer Rechenanordnung (R) versehene Anzeigevorrichtung (A3) vorgesehen ist, mit der von der Ionenstärke abhängige Grössen berechenbar und anzeigbar sind.

17. Anordnung nach einem der Ansprüche 2, 7, 9, 11-16, dadurch gekennzeichnet, dass der erste Indikator für die fluorszenzfotometrische Messung eine möglichst grosse Wertigkeit hat.

**Claims**

1. Method for measuring the ionic strength, characterized by determination of the pH-value of an electrolyte with a first measurement of a first indicator depending on the ionic strength, and with a second measurement of a second indicator depending on the ionic strength, the dependence on the ionic strength of the second indicator being different from the dependence of the first indicator, the ionic strength being calculated by the approximation

$$J = J_0 \cdot 10^{\,F(N)\left[1 + \frac{W(X) + 0,5}{W(N) - W(X)}\right][pH'(N) - pH'(X)]} \tag{2}$$

J = Ionic strength
$J_0$ = Ionic strength by calibration
pH'(N) = pH-value, measured with the first indicator N
pH'(X) = pH-value, measured with the second indicator X
W(N) = valence of the first Indicator
W(X) = valence of the second Indicator

with

$$F(N) = -\frac{1}{0,07(2\ W(N) + 1)} \tag{2a}$$

2. Apparatus for using the method of claim 1, characterized by a first arrangement for the determination of the pH-value with a first indicator depending on the ionic strength and by a second arrangement for the determination of the pH-value with a second indicator the dependence on the ionic strength of the second indicator being different from that of the first indicator, the difference of the signals (d1) being the power in a network for raising to power (P1, P2) the parameter

$$E_1 = J_0 \cdot 10^{\,F(N)\left[1 + \frac{W(X) + 0,5}{W(N) - W(X)}\right]}$$

to

$$(E_1)^{d_1} = J_0^{d_1} \cdot 10^{\,F(N)\left[1 + \frac{W(X) + 0,5}{W(N) - W(X)}\right] \cdot d_1}$$

with

$$F(N) = -\frac{1}{0,07(2\ W(N) + 1)}$$

and that the signal is fed to a measuring instrument.

3. Method according to claim 1, characterized by the indicators being fluorescent indicators with different dependencies on ionic strength.

4. Method according to claim 3, characterized by the indictors bein hydroxypyrentrisulfonat (HPTS) and b-Methylumbelliferon (b-M).

5. Method according to claim 3, characterized by a reference indicator being added to the indicators for the elimination of fluctuations of intensity.

6. Method according to claim 1, characterized by one of the indicators being a pH-electrode and the ionic strength being calculated according to

$$J = J_0 \cdot 10^{2,22[pH'(N) - pH]} \tag{4}$$

9

pH'(N) = pH-value with first indicator
pH = pH-value with pH-electrode

7. Apparatus for using the Method of claim 6, characterized by a first arrangement for measuring the pH-value by fluorescence (I1, F1) and by a second arrangement for measuring the pH-value with a pH-electrode (E), the difference (d1) of the signals being the power in a network for raising to power (P2) the parameter

$$E_2 = J_0 10^{2,22} \tag{5}$$

to

$$E_2^{d1} = J_0^{d1} \cdot 10^{2,22 \cdot d1} \tag{5b}$$

and the signal is fed to a measuring instrument.

8. Method for measuring the pH-value of an unknown electrolytic solution by fluorescence, characterized in that the signal difference of the signal as measured with a first indicator (X) and the signal as measured with the second indicator (N) is multiplied with a factor of valence (WF) and subtracted from the signal of the first indicator so that

$$pH = pH' - WF[pH'(N) - pH'(X)] \tag{21}$$

pH'(N) = virtual pH(N)
pH'(X) = virtual pH(X)
pH = actual pH
WF = factor of valence

with

$$WF = \frac{W(X) + 0,5}{W(N) - W(X)} \tag{21a}$$

W = valence

9. Apparatus for using the method according to claim 8, characterized by a network for subtraction which is subtracting the signal difference (d2) of the signal of the first indicator and of the signal of the second indicator, multiplied with a factor of valence (WF), from the signal of the first indicator the factor of valence being

$$WF = \frac{W(X) + 0,5}{W(N) - W(X)} \tag{21a}$$

and the resulting signal being fed to measuring instrument.

10. Method for measuring the pH-value by fluorescence according to claim 8, characterized by feeding the signals to a real-time computer and an indicator.

11. Apparatus according to claim 7, characterized by feeding the signal of the pH-electrode to an additional measuring instrument (A2).

12. Apparatus according to claim 2 or 7, characterized by an additional instrument (A3) for calculating and indicating parameters depending on the ionic strength.

13. Apparatus according to claim 12, characterized by the formula

$$Osm = 2J$$

being the calculating characteristic of the instrument.

14. Apparatus according to claim 12, characterized by the formula

$$P_0 = 2J \cdot 22,4$$

being the calculating characteristic of the instrument.

15. Apparatus according to claim 9, characterized by the signal difference (d2) being the power for the network for raising to power (P1) the parameter

$$J_0 \cdot 10^{2,22}$$

to

$$J_1 = J_0^{d2} \cdot 10^{2,22 \cdot d2}$$

and that the signal is fed to a measuring instrument (A3).

16. Apparatus according to claim 15, characterized by an additional computer (R) having a indicating instrument (A3) for computation and indication of values depending on ionic strength.

17. Apparatus according to one of the claims 2, 7, 9, 11-16, characterized by a first fluorescence indicator with a valence as large as possible.

## Revendications

1. Méthode pour mesurer la force ionique, caractérisée par la détermination de la valeur de pH d'une électrolyte avec une mesure première d'un indicateur premier dépendant de la force ionique, et avec une mesure seconde avec un indicateur second, la dépendance de la force ionique de l'indicateur second étant différent de l'indicateur premier, la force ionique étant calculée par l'approximation

$$J = J_0 \cdot 10^{\,F(N)\left[1 + \frac{W(X) + 0,5}{W(N) - W(X)}\right][pH'(N) - pH'(X)]} \tag{2}$$

$J$ = force ionique
$J_0$ = force ionique par calibration
$pH'(N)$ = valeur de pH, mesure par l'indicateur premier (N)
$pH'(X)$ = valeur de pH, mesure par l'indicateur second (X)
$W(N)$ = valence de l'indicateur premier
$W(X)$ = valence de l'indicateur second

avec

$$F(N) = -\frac{1}{0,07(2\,W(N) - 1)} \tag{2a}$$

2. Appareil pour l'utilisation de méthode de la revendication 1, caractérisé par un arrangement premier avec un indicateur premier dépendant de la force ionique et un arrangement second avec un indicateur second dépendant de la force ionique en manière différente de l'indicateur premier, la différence des signaux (d1) étant la puissance pour un réseau à élever à une puissance (P1, P2) le paramètre

$$E_1 = J_0 \cdot 10^{\,F(N)\left[1 + \frac{W(X) + 0.5}{W(N) - W(X)}\right]}$$

à

$$(E_1)^{d_1} = J_0^{d_1} \cdot 10^{\,F(N)\left[1 + \frac{W(X) + 0.5}{W(N) - W(X)}\right] \cdot d_1}$$

avec

$$F(N) = -\frac{1}{0,07(2\,W(N) + 1)}$$

et ce signal est raccordé avec un appareil d'indication.

3. Méthode selon la revendication 1, caractérisée par les indicateurs étant indicateurs de fluorescence avec des dépendances différentes de la force ionique.

4. Méthode selon la revendication 3, caractérisée par les indicateurs étant hydrogenpyrentrisulfonate (HPTS) et b-méthylumbelliféron (b-M).

5. Méthode selon la revendication 3, caractérisée par l'addition d'un indicateur de référence pour l'élimination des fluctuations d'intensité.

6. Méthode selon la revendication 1, caractérisée par l'un des indicateurs étant un électrode pour mesurer le pH, la force ionique est calculée selon

$$J = J_0 \cdot 10^{2,22[pH'(N) - pH]} \tag{4}$$

pH'(N) = pH avec indicateur premier
pH = pH avec pH-électrode.

7. Appareil pour l'utilisation de méthode de la revendication 6, caractérisé par un arrangement premier pour mesurer la valeur pH avec la fluorescence (I1, F1) et par un arrangement second pour mesurer la valeur pH avec un électrode (E), la différence (d1) des signaux étant la puissance pour un réseau à élever à une puissance (P2) le paramètre

$$E_2 = J_0 \cdot 10^{2,22} \tag{5}$$

à

$$E_2^{d1} = J_0^{d1} \cdot 10^{2,22 \cdot d1} \tag{5b}$$

et ce signal est raccordé avec un appareil d'indication.

8. Méthode pour mesurer la valeur pH d'une solution électrolyte inconnue par la fluorescence, caractérisée par la différence des signaux mesurée avec l'indicateur premier (X) et avec l'indicateur second (N) et multipliée avec un facteur du valence (WF) et déduite du signal d'indicateur premier selon

$$pH = pH' - WF[pH'(N) - pH'(X)] \tag{21}$$

pH'(N) = pH(N) virtuelle
pH'(X) = pH(X) virtuelle
pH = pH actuelle
WF = facteur de valence
W = valence

avec

$$WF = \frac{W(X) + 0,5}{W(N) - W(X)} \cdot$$

9. Appareil pour l'utilisation de méthode selon la revendication 8, caractérisé par un réseau de déduction pour déduire la différence (d2) des signaux de l'indicateur premier et de l'indicateur second, multiplié avec un facteur de valence (WF), du signal d'indicateur premier, le facteur de valence étant

$$WF = \frac{W(X) + 0,5}{W(N) - W(X)} \cdot \tag{21a}$$

et ce signal est raccordé avec un appareil d'indication.

10. Méthode pour mesurer la valeur pH par fluorescence selon la revendication 8, caractérisée par raccordement des signaux avec un calculateur immédiat et un appareil d'indication.

11. Appareil selon la revendication 7, caractérisé par raccordement du signal de pH = électrode avec un appareil d'indication additionnel.

12. Appareil selon les revendications 2 ou 7, caractérisé par un instrument additionnel (A3) pour calculer et indiquer les paramètres dépendant de force ionique.

13. Appareil selon la revendication 12, caractérisé par la formule

$$Osm = 2J$$

étant la caractéristique de calculation de l'instrument.

14. Appareil selon la revendication 12, caractérisé par la formule

$$P_0 = 2J \cdot 22,4$$

étant la caractéristique de calculation de l'instrument.

15. Appareil selon la revendication 9, caractérisé par la différence des signaux (d2) étant la puissance pour un réseau à élever à une puissance (P1) le paramètre

$$J_0 \cdot 10^{2,22}$$

à

$$J_1 = J_0^{d2} \cdot 10^{2,22 \cdot d2}$$

et ce signal est raccordé avec un appareil d'indication (A3).

16. Appareil selon la revendication 15, caractérisé par un calculateur additionnel (R) avec un instrument (A3) pour calculation et pour indication de valeurs dépendants de force ionique.

17. Appareil selon les revendications 2, 7, 9, 11-16, caractérisé par un indicateur de fluorescence avec une valence la plus grande.

FIG.1

FIG.2

FIG.3

FIG.4

2